# EUROPÄISCHE PATENTSCHRIFT

(11) **EP 0 979 069 B1**
(45) Veröffentlichungstag und Bekanntmachung des Hinweises auf die Patenterteilung: **12.01.2005**
(21) Anmeldenummer: 98928152.2
(22) Anmeldetag: 07.04.1998
(51) Int. Cl.: A61K 9/48

(54) **VERFAHREN ZUR HERSTELLUNG PERORAL ANWENDBARER FESTER ARZNEIFORMEN MIT GESTEUERTER WIRKSTOFFABGABE**
METHOD FOR PRODUCING ORALLY ADMINISTERED, SOLID PHARMACEUTICAL PRODUCTS WITH CONTROLLED RELEASE OF THE ACTIVE SUBSTANCE
PROCEDE POUR PRODUIRE DES FORMES GALENIQUES SOLIDES POUVANT ETRE ADMINISTREES PAR VOIE ORALE, A LIBERATION DE PRINCIPE ACTIF CONTROLEE

(30) Priorität: 29.04.1997 DE 19718012
(43) Veröffentlichungstag der Anmeldung: 16.02.2000
(73) Patentinhaber: Schering AG, 13353 Berlin (DE)
(72) Erfinder: DITTGEN, Michael, D-99510 Apolda (DE); FRICKE, Sabine, D-07749 Jena (DE); TIMPE, Carsten, D-37276 Meinhard (DE); GERECKE, Hagen, D-07743 Jena (DE); EICHARDT, Annette, D-07616 Bürgel (DE)
(74) Vertreter: Cramer, Eva-Maria
(86) Internationale Anmeldenummer: PCT/DE1998/000979
(87) Internationale Veröffentlichungsnummer: WO 1998/048782

(56) Entgegenhaltungen:
- EP-A- 0 548 595
- EP-A- 0 722 732
- WO-A-92/00730
- WO-A-92/10173
- WO-A-98/25613
- WO-A-98/29095
- DE-A- 2 749 745

## Beschreibung

Die Erfindung betrifft ein Verfahren zur Herstellung peroraler anwendbarer fester Arzneiformen mit gesteuerter Wirkstoffabgabe, wobei mindestens drei von vier einen Wirkstoff oder eine Wirkstoffkombination enthaltende Komprimate in ihrer Auswahl und Anzahl variierbar sind und nach bekannten, mit geringem apparativen und zeitlichen Aufwand beherrschbaren Verfahren zu festen Arzneiformen verarbeitet werden. Durch dieses Verfahren werden perorale feste Arzneiformen hergestellt, die die verschiedenartigsten pharmazeutisch erforderlichen Freisetzungsprofile von Wirkstoffen oder Wirkstoffkombinationen, wie beispielsweise eine verzögerte, eine gleichmäßig hinhaltende oder eine an speziellle Rhytmen angepaßte (pulsatile) Freisetzung, realisieren. Unter Freisetzungsprofil ist nachfolgend die Freisetzung (Abgabe) des Wirkstoffes oder Wirkstoffkombination aus der Arzneiform in Abhängigkeit von der Zeit zu verstehen.

Ziel in der pharmazeutischen Technologie ist es, Verfahren zur Überführung eines pharmazeutischen Wirkstoffes in eine Arzneiform zu finden, die u.a. eine therapiegerechte Freigabe des Wirkstoffes nach Applikation gewährleistet.

Dabei soll am Wirkungsort ein für die Therapie optimaler Konzentrationsverlauf erreicht werden.

Ideal, jedoch nur in den seltensten Fällen anwendbar, sind Mechanismen, bei denen die Wirkstofffreigabe von der tatsächlich am Wirkungsort vorliegenden Wirkstoffkonzentration oder von einer den Krankheitszustand charakterisierenden biochemischen Größe direkt geregelt wird.

Man ist daher auf eine gesteuerte Wirkstoffabgabe angewiesen.

Bekannte Verfahren und Techniken zur Herstellung fester peroral anwendbarer Arzneiformen mit gesteuerter Wirkstoffabgabe zielen auf eine verzögerte Freisetzung des Wirkstoffes oder eine gleichmäßig hinhaltende Freisetzung oder auf eine speziellen Rhytmen angepaßte (pulsatile) Freisetzung des Wirkstoffes.

Denkbar wäre auch eine gleichmäßig hinhaltende Freisetzung nachfolgend einer raschen Freisetzung einer Initialdosis des Wirkstoffs.
Ein Hersteller solcher Arzneiformen muß daher mehrere solcher Verfahren und Techniken anwenden, die Ausrüstungen hierfür vorrätig halten, mehrere meist spezielle Hilfsstoffe, die für die einzelnen Verfahren gebraucht werden, beziehen und analysieren.

Aus der Fach- und Patentliteratur sind Verfahren und Techniken zur Herstellung fester peroral anwendbarer Arzneiformen mit gesteuerter Wirkstoffabgabe bekannt, die auf der Basis eines Steuerungsprinzips mehrere der genannten Freisetzungsprofile realisieren sollen.

Problematisch bei peroralen festen Arzneiformen ( 'Single-unit'-Form) mit einer längeren Verweilzeit als etwa 1 h im Magen-Darm-Trakt nach MUTSCHLER, E. et al., 'Arzneimittelwirkungen', Lehrbuch der Pharmakologie und Toxikologie, 7. Aufl., Wissenschaftliche Verlagsgesellschaft mbH Stuttgart 1996, S. 12, jedoch ist, daß die Arzneiform aufgrund der Größe oder Beschaffenheit bestimmte Stellen nicht passieren kann. Eine magensaftresistent überzogene große Tablette läuft beispielsweise Gefahr, den Magen nicht verlassen zu können.
Nach BAUER, K. H. et al., Pharmazeutische Technologie, 4. Auflage, Georg Thieme Verlag Stuttgart, New York, 1993, S. 357, weisen retardierte 'Single-unit'-Formen jedoch den Vorteil einer homogenen Matrix auf.
Bei der 'Multiple-unit'-Form zerfällt die Arzneiform meist im Magen in mehrere Untereinheiten, Granulatkörner oder Pellets.
Ein Nachteil bei der Herstellung von 'Multiple-unit'-Formen besteht darin:
Pellets und Granulate werden volumendosiert in eine Kapsel abgefüllt. Der Fehler der Abfüllung beträgt damit mindestens ein Pellet oder ein Granulatkorn.
Daneben werden Minitabletten als Füllung in Kapseln verwendet. Minitabletten erfordern spezielle Preßwerkzeuge und stellen sehr hohe Ansprüche an die Rezeptur.

Auch NÜRNBERG, E. und SURMANN, P. (Hrsg.) weisen in HAGERS HANDBUCH, Bd. 2 -METHODEN, 5. vollst. neubearb. Auf., Springer Verlag Berlin, Heidelberg, New York, 1991, S. 1123, im Hinblick auf die Magenpassagezeit- bedingt durch die jeweilige Arzneiform - auf die deutlichen Unterschiede zwischen den monolithischen 'Single-unit'-Formen und den 'Multiple-unit'-Formen hin.

Die die Wirkstoffabgabe steuernden Größen basieren in den meisten Fällen auf osmotischen Vorgängen, auf einen Diffusionsprozeß und/oder Erosionsprozeß.
Nach OURIEMCHI, E.M. et al, 'Oral Dosage Forms with a Core and Shell with the Same Polymer Containing Different Drug Concentrations', Int. J. Pharm., 102 (1994), S. 47-54, wirken in Arzneiformen mit gesteuerter Wirkstoffabgabe, beeinflußt beispielsweise durch eine monolithische Matrix, quellfähige Hydrogelmatrix oder Überzüge, mehrere der genannten Prinzipien gemeinsam.

DILUCCIO, R.C. et al., 'Sustained-Release Oral Delivery of Theophylline by Use of Polyvinyl Alcohol and Polyvinyl Alcohol-Methyl Acrylate Polymers', J. Pharm. Scien., 83 (1994) S. 104-106, beschreiben die gesteuerte Wirkstoffabgabe aus Arzneiformen in Form einer verzögerten Freisetzung des Wirkstoffes Theophyllin durch Verwendung einer Mischung aus Polyvinylalkohol (PVA) und einem Polyvinylalkohol-Methylacrylat-Copolymer (PVA-MA) mit niedriger Kristallinität.
Während PVA allein zu schnell freisetzenden Tabletten führt, ermöglicht PVA-MA je nach Anteil eine verzögerte Wirkstofffreisetzung. Es wurde gefunden, daß eine Mischung PVA : PVA-MA von 1:9:10 die erwünschte verzögerte Freisetzung des Wirkstoffes bewirkt, so daß die Tabletten in einem Test am Hund eine Bioverfügbarkeit von nahezu 80% erreichten.

Auch MESHALI, M.M. et al., 'Preparation and evaluation of theophylline sustained-release tablets', Drug Develop. Ind. Pharm., 22 (1996), S. 373-376 zeigen die verzögerte Freisetzung von Theophyllin, basierend auf der Kombination verschiedener Polyacrylate, die eine gemischte Barriere (mixed barrier) um die Arzneiform legen und zu einer Matrix-Diffusions-gesteuerten Freisetzung des Wirkstoffs führen.
Die Arzneiformen enthalten 50% Theophyllin, Carbopol® 974P als retardierenden Zusatz, sprühgetrocknete Lactose und 0.5% Gleitmittel. Es ließen sich mit diesem Prinzip nur zwei der üblichen Freisetzungsprofile realisieren, eine Freisetzung 0. Ordnung - Wirkstoffabgabe verläuft weitgehend linear, d.h.im gleichen Zeitintervall werden gleiche Wirkstoffmengen abgegeben - und eine Freisetzung proportional zur Quadratwurzel der Zeit.

JUNGINGER, H.E., 'Oral Applications of Pulsatile Drug Delivery' beschreibt in: Gurny, R., Junginger, H.E., Peppas, N.A. (Hrsg.), Pulsatile Drug Delivery, Ed. 1, Wissenschaftliche Verlagsgesellschaft mbH, Stuttgart, 1993, S. 113-134, mehrere Systeme zur Erzielung von pulsatilen Freisetzungsprofilen der Wirkstoffabgabe, wie überzogene Tabletten, Pellets oder Mikrokügelchen, OROS® , PULSINCAP® ; zeitkontrollierte Explosionssysteme, spezielle Schichttabletten.
Die Herstellung dieser Arzneiformen ist grundsätzlich aufwendig und die einwandfreie Funktion der Systeme hängt zumeist entscheidend von der exakten Einhaltung bestimmter Produktionsparameter ab, wie von der Überzugsschichtdicke, der Präzision der Freigabeöffnung und/oder dem Überzug, der Hydrogelbeschaffenheit der genauen Dimensionierung und gegebenfalls der Alterung des Gels, der exakten Abstimmung zwischen osmotischen Kernmaterial und äußerem Überzug und der Präzision der Pressung und der Genauigkeit der erzeugten Schichdicke. Diese Verfahren beinhalten somit alle den erheblichen Nachteil, daß spezielle Fertigungslinien, sowie aufwendige Vorrichtungen oder eine kostenintensive Präzisionsfertigung notwendig sind.

In der Patentschrift DE 44 43 175 A1 wird eine pulsatile Arzneiform aufgezeigt, deren Wirkstofffreigabe den Krankheits- oder Schmerzschüben angepaßt ist.
Die Patentschrift EP 0 719 555 A2 beschreibt die Verwendung von Melatonin zur Herstellung peroraler pulsatiler Arzneiformen.

Die dabei vorgeschlagene pulsatile Arzneiform ist eine Kapsel. Diese enthält den Wirkstoff in verschiedene Trägerstoffe eingebettet. Es sind jedoch keine Komprimate speziell definiert worden, die den Wirkstoff in spezifischen Anteilen freisetzen. Weiterhin wird diese pulsatile Arzneiform nicht für Wirkstoffkombinationen beansprucht.
Aus den Beispielen zu DE 44 43 175 A2 ist zu ersehen, daß der Wirkstoff Melatonin in Kollagenkügelchen eingebettet wurde. Diese Kollagenkügelchen wurden mit einer weiteren Menge Melatonin in Erdnußöl dispergiert. Die Abfüllung eines solchen multidispersen öligen Systems in Kapseln erfordert besonderes technisches Know-how und appparative Voraussetzungen, die den meisten pharmazeutischen Herstellern nicht zugänglich sind.

In WO 92 101 73 A wird eine multipartikuläre perorale Zubereitung von HZ-Antagonisten, vorzugsweise Cimetidin, beschrieben. In den aufgeführten Beispielen werden Pelletmischungen in unterschiedlichen Mengen verhältnissen verwendet. Komprimate in Kapseln können dagegen aufgrund ihrer Größe nur Verhältnisse von 1:1:1(:1) darstellen.

In WO 98/29095 wird ein Applikations problem des Wirkstoffes Cisaprid gelöst. Dazu werden Mischungen von Minitabletten in Kapseln abgefüllt. Derartige Mischungen von multiple unit dosage forms (vorzugsweise Pellets, Minitabletten, Granulate) sind bekanntes Prinzip zur Einstellung von Freisetzungsprofilen.

Der Erfindung liegt die Aufgabe zugrunde, ein Verfahren zur Herstellung peroral anwendbarer fester Arzneiformen mit gesteuerter Wirkstoffabgabe zu schaffen, wobei jedes pharmazeutisch erforderliche Freisetzungsprofil realisiert werden kann und die Herstellung der Arzneiformen mit geringem apparativen und zeitlichen Aufwand erfolgt.

Die Aufgabe wird erfindungsgemäß dadurch gelöst, dass ein Verfahren zur Herstellung peroral anwendbarer tester, als Wirkstoff oder Wirkstoffkombination hormonelle Wirkstoffe enthaltende Arzneiformen mit gesteuerter Wirkstoffabgabe, dadurch gekennzeichnet, daß
a) man mindestens drei der vier unter b) aufgeführten, den Wirkstoff oder die Wirkstoffkombination enthaltende Komprimaten kombiniert, wobei diese nach bekannten Verfahren durch Mischen von Wirkstoffen mit pharmazentisch verträglichen Hilfsstoffen und/oder Trägerstoffen, Granulieren, Tablettieren und/oder Überzichen hergestellt werden,
b) dadurch das pharmazeutisch erforderliche Freisekungsprofil des Wirkstoffes oder der Wirkstoffkombination, erzeugt wird, wobei
- ein Komprimat A der vier Komprimate mindestens 75% seines Wirkstoffgehaltes innerhalb von 45 min,
- ein weiteres Komprimat B 100% seines Wirkstoffgehaltes frühestens nach 3h nach einem Freisetzungsprofil annähernd O. Ordnung, erhältlich mit hydrophilen, lipophilen Matrix tabletten oder diffusions gesteuerten Lacküberzügen,
- ein drittes Komprimat C bei einem pH-Wert von 6-7,5 mindestens 75% seines Wirkstoffgehaltes innerhalb 45 min, erhältlich analog einem Komprimat A miteinem magensaffresistenten überzug,
- ein Komprimat D bei einem pH-Wert von 6-7,5 100% seines Wirkstoffgehaltes frühestens nach 3h nach einem Freisetzungsprofil O. Ordnung, erhältlich mit magensaftresistenten Matrixtabletten oder Kombinationen von magensaftresistenten mit diffusions gesteuerten Lacküberzügen, treisetzt,
wobei das Freisetzungsprofil folgender maßen bestimmt wird:

| | |
|---|---|
| Apparatur | gemäß DAB 96, V.5.4. |
| Rührgeschwindigkeit | 100 U/min ± 2 U/min |
| Temperatur | 37°C ± 0,5 K |
| Mediumvolumen | 1000 ml |
| Medium | 0,1 N HCl, 0,2 M Trinatrium-phosphatpuffer nach USP 23<724>, Methode A. |

eingesetzt wird.

Eine vorteilhafte Ausgestaltung der Erfindung wird in Anspruch 2 aufgezeigt.
Die nach diesem Verfahren hergestellte Kapsel weist die Vorteile einer 'Multiple-unit'-Form auf.

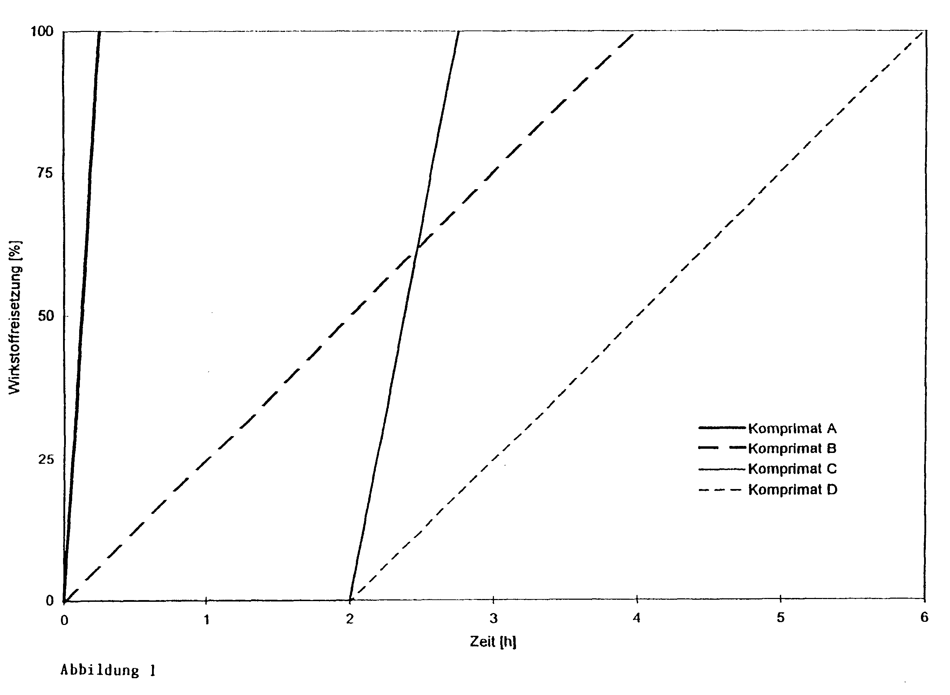

Abbildung 1 zeigt das Freisetzungsprofil der Wirkstoffe anhand der Komprimate A bis D als Modellform auf.
Es wird die Abhängigkeit der prozentualen Wirkstofffreisetzung von der Zeit dargestellt.
Durch Variation der Komprimate in Auswahl und Anzahl sind alle gewünschten Freisetzungsprofile realisierbar.

Mit der Erfindung wird die Applikation von körpereigenen Hormonen und deren verwandten Substanzen außerordentlich gut ermöglicht.
Ein Teil der körpereigenen Hormone zeichnen sich, bedingt durch ihren Metabolismus, durch eine kurze Verweildauer im Körper aus. Beispiele solcher Hormone sind: Progesteron, Testosteron, Dehydroepiandrosteron, Estriol, Estradiol.
Der Spiegel anderer körpereigener Hormone folgt einem ausgeprägten cirkadianen Rhythmus, d.h. ihre Konzentration im Blut ändert sich im Verlauf von 24 h. Beispiele dieser Hormone sind: Prednison, Prednisolon, Cortexon, Corticosteron, Cortisol und Aldosteron. Melatonin wird beispielsweise vorwiegend während der Nachtstunden sezerniert.

Um einen konstanten, der therapeutischen Zielsetzung entsprechenden Wirkstoffspiegel zu garantieren, müsste in all diesen Fällen häufig nachdosiert werden.

Hormonelle Wirkstoffe können auch in Kombinationen angewendet werden. Dabei kann es erforderlich sein, jeden hormonellen Wirkstoff entweder mit einem eigenen Freisetzungsprofil oder gemeinsam in einem Freisetzungsprofil zu applizieren. Beispiele solcher Kombinationen hormoneller Wirkstoffe können sein: Progesteron/Estradiol, Testosteron/Progesteron, Progesteron/Estriol, Progesteron/Estron, Cortisol/Aldosteron.

Mit dem erfindungsgemäßen Verfahren werden peroral anwendbare feste Arzneiformen hergestellt, die alle denkbaren pharmazeutisch erforderlichen Freisetzungsprofile von Wirkstoffen oder Wirkstoffkombinationen realisieren und deren Herstellung mit wenigen gut beherrschbaren technologischen Verfahren und geringen apparativen und zeitlichem Aufwand möglich ist.

Die Erfindung wird durch die nachfolgenden Beispiele näher erläutert.

### Wirkstofffreisetzung:

Die Wirkstofffreistzung bzw. die Freisetzung der Wirkstoffkombination wird in vitro, wie folgt, untersucht:

| | |
|---|---|
| Apparatur | gemäß DAB 96, V.5.4. |
| Rührgeschwindigkeit | 100 U/min ± 2 U/min |
| Temperatur | 37°C ± 0,5 K |
| Mediumvolumen | 1000 ml |
| Medium | 0,1 N HCl, 0,2 M Trinatriumphosphatpuffer nach USP 23 <724>, Methode A |

### Beispiel 1

### Progesteron 50 mg, Estradiol 1,6 mg Zielstellung zum Freisetzungsprofil:

- gepulste Freisetzung:
   - Progesteron: - wird in einer Initialdosos von 10 mg schnell,
   - danach gleichmäßig hinhaltend und
   - Estradiol: - wird schnell abgegeben.

| **Rezeptur:** | **Komprimat A** | **Komprimat C** | **Komprimat D** |
|---|---|---|---|
| | **[mg]** | **[mg]** | **[mg]** |
| **Progsteron** | 10 | 10 | 30 |
| **Estradiol** | 1,6 | | |
| Hydroxypropylmethylcellulose Typ 2208 | | | 10 |
| Laktose | 20,9 | 22,5 | 9 |
| Maisstärke | 12 | 12 | 5,5 |
| PVP K25 | 2 | 2 | 2 |
| Magnesiumstearat | 0,5 | 0,5 | 0,5 |
| Talkum | 1,5 | 1,5 | 1,5 |
| Wasser (als TV) | 1,5 | 1,5 | 1,5 |
| *Tablette* | 50 mg, *⌀ 5 mm* | 50 *mg, ⌀ 5 mm* | *60 mg*, *⌀ 5* mm |
| **Überzug:** | ohne | | |
| Eudragit L 30 D | | 7,5 | 7,5 |
| (als LTS) | | | |
| Talkum | | 1,75 | 1,75 |
| Triethylcitrat | | 0,75 | 0,75 |

### Herstellung

Progesteron, Estradiol, Laktose, Maisstärke und Hydroxypropylmethylcellulose werden gemischt und mit einer Lösung des PVP in Ethanol 96 % granulliert. Das Granulat wird getrocknet, mit Talkum und Magnesiumstearat gemischt und zu einer Tablette des angegebenen Durchmesses und der angegebenen Masse verpreßt.
Der Überzug wird auf die Tabletten in einem geeigneten Gerät mittels einer Dispersion von Eudragit, Talkum und Triethylcitrat aufgebracht und getrocknet.
Die so hergestellten Komprimate werden in eine Kapsel eingebracht.

**Tabelle 1**

| In vitro-Freisetzung der Wirkstoffe (kumuliert): | | | | | | |
|---|---|---|---|---|---|---|
| Zeit [h] | Komprimat A | | Komprimat C | Komprimat D | Arzneiform | gesamt |
| | [mg Progesteron] | [mg Estradiol] | [mg Progesteron] | [mg Progesteron] | [mg Progesteron] | [mg Estradiol] |
| 0,25 | **10** | **1,6** | 0 | 0 | 10 | **1,6** |
| 1 | 10 | 1,6 | 0 | 0 | 10,0 | 1,6 |
| 2 | 10 | 1,6 | 0 | 0 | 10,0 | 1,6 |
| 2,5 | 10 | 1,6 | **10** | 3,2 | 23,2 | 1,6 |
| 3 | 10 | 1,6 | 10 | 7,0 | 27,0 | 1,6 |
| 4 | 10 | 1,6 | 10 | 15,2 | 35,2 | 1,6 |
| 5 | 10 | 1,6 | 10 | 23,8 | 43,8 | 1,6 |
| 6 | 10 | 1,6 | 10 | **30** | **50** | 1,6 |

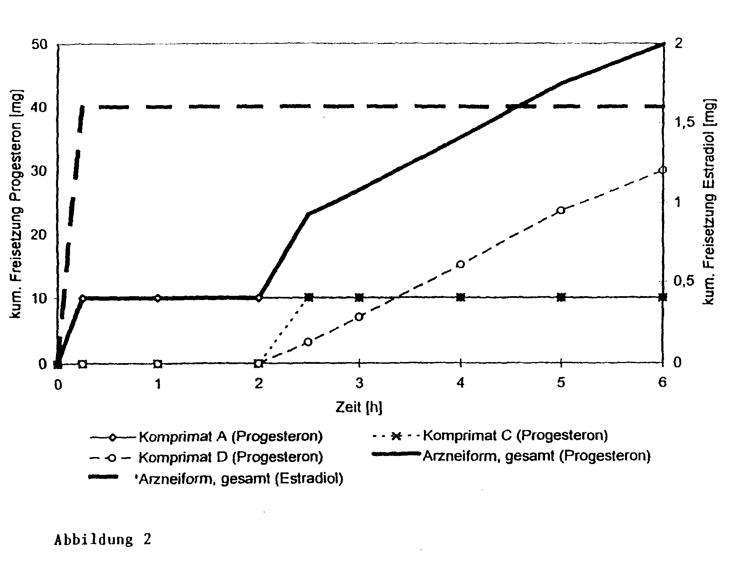

Abbildung 2 in Verbindung mit Tabelle 1 zeigt das Freisetzungsprofil der Wirkstoffe Progesteron, Estradiol als Abhängigkeit ihrer mengenmäßigen Freisetzung von der Zeit.
Drei Komprimate in jeweils einer Ausführung kommen zur Anwendung:
Komprimat A - 10 mg Progesteron, 1,6 mg Estradiol
Komprimat C - 10 mg Progesteron
Komprimat D - 30 mg Progesteron.
Es wird jede Wirkstoffkomponente mit eigenem Freisetzungsprofil appliziert.
Es erfolgt mittels des Komprimates A eine schnelle Wirkstoffabgabe - 10 mg Progesteron und 1,6 mg Estradiol nach 0,25 h - , mittels des Komprimates C eine verzögerte Wirkstoffabgabe - 10 mg Progesteron nach 2,5 h - und mittels des Komprimates D eine Wirkstoffabgabe, welches die verzögernde Momente mit gleichmäßig hinhaltenden Momenten kombiniert - 30 mg Progesteron nach 6 h.

### Beispiel 2

### Melatonin 10 mg Zielstellung zum Freisetzungsprofil:

kumulative Freisetzung von Melatonin,

| **Rezeptur**: | | | |
|---|---|---|---|
| | **Komprimat A** **[mg]** | **Komprimat B** **[mg]** | **Komprimat D** **[mg]** |
| **Melatonin** | 2,5 | 2,5 | 5 |
| HPMC Typ 2208 | | 5 | 5 |
| Laktose | 26,5 | 22,5 | 20 |
| Maisstärke | 15 | 15 | 15 |
| PVP K25 | 1,5 | 1,5 | 1,5 |
| Magnesiumstearat | 0,5 | 0,5 | 0,5 |
| Talkum | 1,5 | 1,5 | 1,5 |
| Croscarmelose-Na | 1 | | |
| Wasser (als TV) | 1,5 | 1,5 | 1,5 |
| *Tablette* | *50 mg, ⌀ 5 mm* | 50 *mg, ⌀ 5 mm* | *50 mg, ⌀ 5 mm* |
| **Überzug:** | ohne | ohne | |
| Eudragit L 30 D | | | 3,76 |
| (als LTS) | | | |
| Talkum | | | 0,81 |
| Glyceroltriacetat | | | 0,38 |
| Antischaumemulsion | | | 0,05 |

### Herstellung

Melatonin, Laktose, Maisstärke und Hydroxypropylmethylcellulose werden gemischt und mit einer Lösung des PVP in Ethanol 96 % granulliert. Das Granulat wird getrocknet, mit Talkum und Magnesiumstearat gemischt und zu einer Tablette des angegebenen Durchmesses und der angegebenen Masse verpreßt.
Der Überzug wird auf die Tabletten in einem geeigneten Gerät mittels einer Dispersion von Eudragit, Talkum, Glyceroltriacetat und Antischaumemulsion aufgebracht und getrocknet.
Die so hergestellten Komprimate werden in eine Kapsel eingebracht.

**Tabelle 2**

| In vitro-Freisetzung des Melatonins (kumuliert): | | | | |
|---|---|---|---|---|
| Zeit [h] | Komprimat A [mg Melatonin] | Komprimat B [mg Melatonin] | Komprimat D [mg Melatonin] | Arzneiform gesamt |
| 0,17 | **2,5** | 0 | 0 | 2,48 |
| 1 | 2,5 | 1,03 | 0 | 3,51 |
| 2 | 2,5 | 1,65 | 0,14 | 4,26 |
| 3 | 2,5 | 2,13 | 2,14 | 6,75 |
| 4 | 2,5 | **2,5** | 4,65 | 9,51 |
| 5 | 2,5 | 2,5 | 4,89 | 9,75 |
| 6 | 2,5 | 2,5 | **5** | **10** |

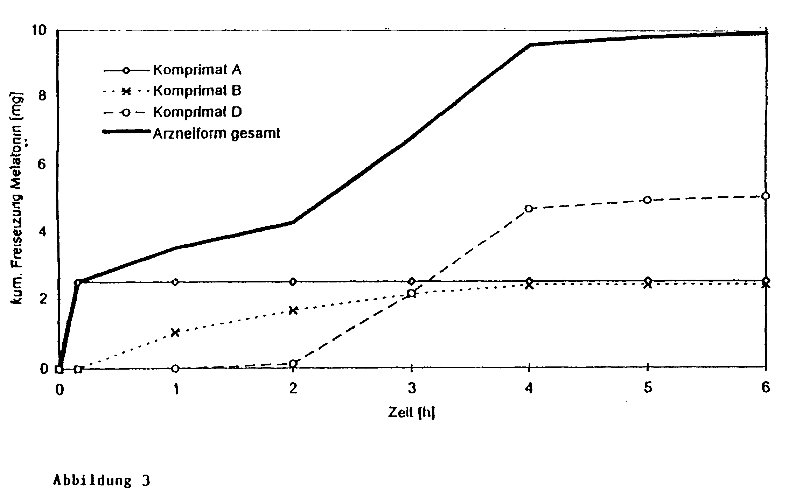

Abbildung 3 in Verbindung mit Tabelle 2 zeigt das Freisetzungsprofil des Wirkstoffes Melatonin als Abhängigkeit der mengenmäßigen Freisetzung von der Zeit.
Drei Komprimate in jeweils einer Ausführung kommen zur Anwendung:
Komprimat A - 2,5 mg Melatonin
Komprimat B - 2,5 mg Melatonin
Komprimat D - 5 mg Melatonin
Es erfolgt mittels des Komprimates A eine schnelle Wirkstoffabgabe - 2,5 mg Melatonin nach 0,17 h - , mittels des Komprimates B eine gleichmäßig hinhaltende Wirkstoffabgabe - 2,5 mg Melatonin nach 4 h - und mittels des Komprimates D eine Wirkstoffabgabe, welche die verzögernden Momente mit gleichmäßig hinhaltenden Momenten kombiniert - 5mg Melatonin nach 6 h.

### Beispiel 3

### Hydrocortison 10 mg Zielstellung zum Freisetzungsprofil:

gleichmäßig hinhaltende Freisetzung mit Verlängerung der Frei setzungszeit

| **Rezeptur**: | | | |
|---|---|---|---|
| | **Komprimat A** **[mg]** | **Komprimat B** **[mg]** | **Komprimat D** **[mg]** |
| **Hydrocortison** | 1 | 3 | 6 |
| HPMC Typ 2208 | | 5 | 5 |
| Laktose | 27 | 22 | 19 |
| Maisstärke | 16 | 15 | 15 |
| PVP K25 | 1,5 | 1,5 | 1,5 |
| Magnesiumstearat | 0,5 | 0,5 | 0,5 |
| Talkum | 1,5 | 1,5 | 1,5 |
| Croscarmelose-Na | 1 | | |
| Wasser (als TV) | 1,5 | 1,5 | 1,5 |
| *Tablette* | *50 mg, ⌀ 5 mm* | *50 mg, ⌀ 5 mm* | *50 mg, ⌀ 5 mm* |
| **Überzug:** | ohne | ohne | |
| Eudragit L 30 D | | | 3,76 |
| (als LTS) | | | |
| Talkum | | | 0,81 |
| Glyceroltriacetat | | | 0,38 |
| Antischaumemulsion | | | 0,05 |

### Herstellung

Hydrocortison, Laktose, Maisstärke und Hydroxypropylmethylcellulose werden gemischt und mit einer Lösung des PVP in Ethanol 96 % granulliert. Das Granulat wird getrocknet, mit Talkum und Magnesiumstearat gemischt und zu einer Tablette des angegebenen Durchmesses und der angegebenen Masse verpreßt.
Der Überzug wird auf die Tabletten in einem geeigneten Gerät mittels einer Dispersion von Eudragit, Talkum, Glyceroltriacetat und Antischaumemulsion aufgebracht und getrocknet.
Die so hergestellten Komprimate werden in eine Kapsel eingebracht.

**Tabelle 3**

| In vitro-Freisetzung des Hydrocortisons (kumuliert): | | | | |
|---|---|---|---|---|
| Zeit [h] | Komprimat A [mg Hydrocortison] | Komprimat B [mg Hydrocortison] | Komprimat D [mg Hydrocortison] | Arzneiform gesamt |
| 0,25 | **1** | 0 | 0 | **1,06** |
| 1 | 1 | 0,9 | 0 | **1,86** |
| 2 | 1 | 2,1 | 0 | **3,06** |
| 2,47 | 1 | 2,3 | 0,8 | **4,13** |
| 3 | 1 | 2,6 | 1,7 | **5,32** |
| 4 | 1 | **3** | 3,1 | **7,07** |
| 5 | 1 | 3 | 4,4 | **8,38** |
| 6 | 1 | 3 | **6** | **10** |

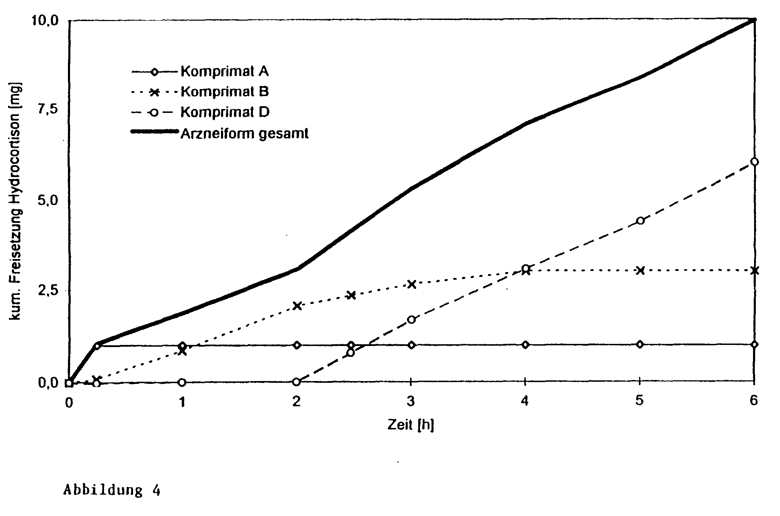

Abbildung 4 in Verbindung mit Tabelle 3 zeigen das Freisetzungsprofil des Wirkstoffes Hydrocortison als Abhängigkeit der mengenmäßigen kumulativen Freisetzung von der Zeit.
Drei Komprimate in jeweiis einer Ausführung kommen zur Anwendung:
Komprimat A - 1 mg Hydrocortison
Komprimat B - 3 mg Hydrocortison
Komprimat D - 6 mg Hydrocortison
Es erfolgt mittels des Komprimates A eine schnelle Wirkstoffabgabe - 1 mg Hydrocortison nach 0,25 h - , mittels des Komprimates B eine gleichmäßig hinhaltende Wirkstoffabgabe - 3 mg Hydrocortison nach 4 h - und mittels des Komprimates D eine Wirkstoffabgabe, welche die verzögernden Momente mit gleichmäßig hinhaltenden Momenten kombiniert - 6 mg Hydrocortison nach 6 h.

## Patentansprüche

1. Verfahren zur Herstellung peroral anwendbarer fester, als Wirkstoff oder Wirkstoffkombination hormonelle Wirkstoffe enthaltende Arzneiformen mit gesteuerter Wirkstoffabgabe, **dadurch gekennzeichnet, daß**
a) man mindestens drei von den vier unter b) aufgeführten, den Wirkstoff oder die Wirkstoffkombination enthaltenden Komprimaten kombiniert, wobei diese nach bekannten Verfahren durch Mischen von Wirkstoffen mit pharmazeutisch verträglichen Hilfsstoffen und/oder Trägerstoffen, Granulieren, Tablettieren und/oder Überziehen hergestellt werden,
b) dadurch das pharmazeutisch erforderliche Freisetzungsprofil des Wirkstoffes oder der Wirkstoffkombination, erzeugt wird,
wobei
- ein Komprimat A der vier Komprimate mindestens 75 % seines Wirkstoffgehaltes innerhalb von 45 min,
- ein weiteres Komprimat B 100 % seines Wirkstoffgehaltes frühestens nach 3 h nach einem Freisetzungsprofil annähernd 0. Ordnung, erhältlich mit hydrophilen, lipophilen Matrixtabletten oder diffusionsgesteuerten Lacküberzügen,
- ein drittes Komprimat C bei einem pH-Wert von 6-7,5 mindestens 75 % seines Wirkstoffgehaltes innerhalb von 45 min, erhältlich analog einem Komprimat A mit einem magensaftresistenten Überzug,
- das vierte Komprimat D bei einem pH-Wert von 6-7,5 100 % seines Wirkstoffgehaltes frühestens nach 3 h nach einem Freisetzungsprofil 0. Ordnung, erhältlich mit magensaftresistenten Matrixtabletten oder Kombinationen von magensaftresistenten mit diffusionsgesteuerten Lacküberzügen, freisetzt,
wobei das Freisetzungsprofil folgender maßen bestimmt wird:
| | |
|---|---|
| Apparatur | gemäß DAB 96, V.5.4. |
| Rührgeschwindigkeit | 100 U/min ± 2 U/min |
| Temperatur | 37°C ± 0,5 K |
| Mediumvolumen | 1000 ml |
| Medium | 0,1 N HCl, 0,2 M Trinatrium-phosphatpuffer nach USP 23<724>, Methode A. |

2. Verfahren zur Herstellung peroral anwendbarer fester Arzneiformen mit gesteuerter Wirkstoffabgabe nach Anspruch 1, **dadurch gekennzeichnet, daß** die perorale feste Arzneiform eine Kapsel ist.

## Claims

1. Process for the preparation of perorally administrable solid pharmaceutical forms having controlled release of active compound, containing, as an active compound or active compound combination, hormonal active compounds, **characterized in that**
a) at least three of the four compressed tablets containing the active compound or the active compound combination mentioned under b) are combined, these being prepared according to known processes by mixing active compounds with pharmaceutically tolerable excipients and/or vehicles, granulating, tabletting and/or coating,
b) the pharmaceutically required release profile of the active compound or of the active compound combination is produced thereby,
- one compressed tablet A of the four compressed tablets releasing at least 75% of its active compound content within 45 min,
- a further compressed tablet B releasing 100% of its active compound content at the earliest after 3 h according to a release profile of approximately 0th order, obtainable using hydrophilic matrix tablets, lipophilic matrix tablets or diffusion-controlled lacquer,coatings,
- a third compressed tablet C releasing, at a pH of 6-7.5, at least 75% of its active compound content within 45 min, obtainable analogously to a compressed tablet A having an enteric coating,
- the fourth compressed tablet D releasing, at a pH of 6-7.5, 100% of its active compound content at the earliest after 3 h according to a release profile of 0th order, obtainable using enteric matrix tablets or combinations of enteric coatings with diffusion-controlled lacquer coatings, the release profile being determined in the following way:
| | |
|---|---|
| Apparatus | according to DAB 96, V. 5.4. |
| Stirring rate | 100 rpm ± 2 rpm |
| Temperature | 37°C ± 0.5 K |
| Volume of medium | 1000 ml |
| Medium | 0.1 N HCl, 0.2 M trisodium phosphate buffer according to USP 23 <724>, method A. |

2. Process for the preparation of perorally administrable solid pharmaceutical forms having controlled release of active compound according to Claim 1, **characterized in that** the peroral solid pharmaceutical form is a capsule.

## Revendications

1. Procédé pour la production de formes galéniques solides pouvant être administrées par voie orale, à libération programmée de substance(s) active(s), contenant des substances actives hormonales en tant que substance active ou association de substances actives, **caractérisé en ce que**
a) on associe au moins trois des quatre comprimés indiqués en b), contenant la substance active ou l'association de substances actives, ces derniers étant fabriqués selon des procédés connus, par mélange de substances actives avec des adjuvants et/ou véhicules pharmaceutiquement acceptables, granulation, transformation en comprimés et/ou enrobage,
b) le profil de libération de la substance active ou de l'association de substances actives, pharmaceutiquement requis, est ainsi produit,
- un comprimé A des quatre comprimés libérant au moins 75 % de sa teneur en substance(s) active(s) en l'espace de 45 minutes,
- un autre comprimé B libérant 100 % de sa teneur en substance(s) active(s) au plus tôt après 3 heures, selon un profil de libération approximativement d'ordre 0, pouvant être obtenu avec des comprimés à matrice hydrophiles, lipophiles ou des enrobages pelliculaires réglés quant à la diffusion,
- un troisième comprimé C libérant à un pH de 6-7,5 au moins 75 % de sa teneur en substance(s) active(s) en l'espace de 45 minutes, pouvant être obtenu de la même façon qu'un comprimé A avec un enrobage résistant au suc gastrique,
- le quatrième comprimé D libérant à un pH de 6-7,5 100 % de sa teneur en substance(s) active(s) au plus tôt après 3 heures selon un profil de libération d'ordre 0, pouvant être obtenu avec des comprimés à matrice résistants au suc gastrique ou des combinaisons d'enrobages résistants au suc gastrique et d'enrobages réglés quant à la diffusion,
le profil de libration étant déterminé de la façon suivante :
| | |
|---|---|
| appareil | selon DAB 96, V. 5.4. |
| vitesse d'agitation | 100 tours/min ± 2 tours/min. |
| température | 37°C ± 0,5 K |
| volume du milieu | 1 000 ml |
| milieu | HCl 0,1 N, tampon phosphate trisodique 0,2 M selon USP 23(724) méthode A. |

2. Procédé pour la production de formes galéniques solides pouvant être administrées par voie orale, à libération programmée de substance(s) active(s) selon la revendication 1, **caractérisé en ce que** la forme galénique solide orale est une gélule.
